(19) Europäisches Patentamt
European Patent Office
Office européen des brevets

(11) Veröffentlichungsnummer: **0 074 497**
**B1**

(12) # EUROPÄISCHE PATENTSCHRIFT

(45) Veröffentlichungstag der Patentschrift: **27.11.85**

(51) Int. Cl.⁴: **C 07 C 69/88**, C 07 C 67/31

(21) Anmeldenummer: **82107318.6**

(22) Anmeldetag: **12.08.82**

(54) **Verfahren zur Herstellung von 3-Alkyl-6-methyl-beta-resorcylsäureestern.**

(30) Priorität: **16.09.81 DE 3136720**

(43) Veröffentlichungstag der Anmeldung: **23.03.83 Patentblatt 83/12**

(45) Bekanntmachung des Hinweises auf die Patenterteilung: **27.11.85 Patentblatt 85/48**

(84) Benannte Vertragsstaaten: **AT BE CH DE FR GB IT LI LU NL SE**

(56) Entgegenhaltungen: **US - A - 4 072 660**

(73) Patentinhaber: **DYNAMIT NOBEL AKTIENGESELLSCHAFT, Postfach 1209, D-5210 Troisdorf, Bez. Köln (DE)**

(72) Erfinder: **Schmidt, Hans-Georg, Dr., Porzer Strasse 15, D-5216 Niederkassel 3 (DE)**

**EP 0 074 497 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung ist ein Verfahren zur Herstellung von 3-Alkyl-6-methyl-β-resorcylsäureester.

β-Resorcylsäureester der Formel I sind Riechstoffe mit «Eichenmooscharacter».

Es sind mehrere Methoden zur Herstellung von alkylsubstituierten β-Resorcylsäureestern aus alkylierten Dihydro-β-resorcylsäureestern bekannt. (DE-OS 2 653 177, BE-PS 7 380 046, NL-PS 807 693.) Die Überführung in den Aromaten erfolgt mit Oxidationsmethoden (z.B. $H_2SO_4$, Halogene). Eine andere Herstellungsmethode besteht in der Umsetzung von Malonestern mit α-,β-ungesättigten-α-Acyloxi-alkenonen in Gegenwart stöchiometrischer Mengen Natriumhydrid (US-PS 3 928 419). In allen Fällen sind die Ausgangsstoffe nicht leicht zugänglich und die Ausbeuten der reinen Endstoffe niedrig. Nach der US-PS 4 072 660 entstehen Resorcylsäureester und Pyrone von anderer Struktur, wobei die Pyrone zudem nur eine Doppelbindung enthalten und eine Dehydrierung zur Bildung der Produkte erforderlich ist.

Der vorliegenden Erfindung liegt die Aufgage zugrunde, ein vereinfachtes Verfahren zu schaffen und leicht zugängliche Ausgangsprodukte zu verwenden.

Gegenstand der Erfindung ist ein Verfahren zur Herstellung von 3-Alkyl-6-methyl-β-resorcylsäureestern der Formel

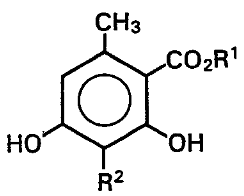

I

das dadurch gekennzeichnet ist, dass man ein α-Pyron der Formel

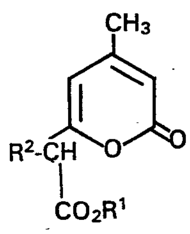

II

in der $R^1$ und $R^2$ gleich oder verschieden sein können und für Alkylreste mit 1 bis 10 C-Atomen stehen, mit einer Base umsetzt.

Bevorzugte Alkylgruppen sind solche mit 1 bis 5 C-Atomen, besonders Methyl, Ethyl, n- und i-Propyl, n- und i-Butyl.

Als Basen werden bevorzugt Alkali- oder Erdalkalialkoholate, Alkali- oder Erdalkalihydroxide oder -carbonate eingesetzt. In der Regel fällt zunächst ein der Formel I entsprechendes Alkali- oder Erdalkali-Resorcinat an, aus dem durch Ansäuern der freie 3-Alkyl-6-methyl-β-resorcylsäureester der Formel I erhalten wird.

Die Base wird vorzugsweise in stöchiometrischen oder geringfügig überstöchiometrischen Mengen eingesetzt.

Die Reaktion wird bevorzugt in einem Lösungsmittel wie z.B. Alkoholen, Äthern usw. durchgeführt. Die Umsetzung lässt sich in einem weiten Temperaturbereich von +10°C bis 200°C vornehmen. Bevorzugt ist die Reaktion im siedenden Lösungsmittel bei Normaldruck oder Eigendruck.

Das Freisetzen der Resorcine der allgemeinen Formel I aus den entsprechenden Resorcinaten kann mit beispielsweise der äquivalenten Menge wässriger Mineralsäuren oder organischer Säuren, wie z.B. Essigsäure, erfolgen. Die Isolierung der Resorcine der allgemeinen Formel I erfolgt dann vorzugsweise durch Extraktion, die Reinigung durch Umkristallisation.

Die Herstellung der Ausgangsverbindungen der allgemeinen Formel II ist in der DE-OS 2 916 648 beschrieben.

Die vorliegende Erfindung wird an den folgenden Beispielen erläutert.

*Beispiel 1*

30 g 4-Methyl-α-pyronyl-6-(α-iso-propyl)-essigsäuremethylester werden in 150 ml Methanol gelöst und diese Mischung mit 10,8 g Natriummethylat versetzt. Unter einer Inertgasatmosphäre wird 3 Stunden am Rückfluss gekocht und nach dem Erkalten der Lösung diese mit 2n wässriger $H_2SO_4$ angesäuert. Durch Extraktion mit Äther erhält man 24,3 g 3-iso-Propyl-6-methyl-β-resorcylsäuremethylester. Nach Umkristallisation aus Chloroform liegt dieser in farblosen Kristallen vor. (Fp. 144-145°C.)

*Beispiel 2*

12 g 4-Methyl-α-pyronyl-6-(α-methyl)-essigsäuremethylester werden mit 5,0 g Na-methylat in 20 ml Methanol versetzt und diese Mischung 3 Stunden am Rückfluss gekocht. Im folgenden wird wie im Beispiel 1 verfahren. Man erhält 9,6 g 3,6-Dimethyl-β--resorcylsäuremethylester, der nach Umkristallisation aus Wasser einen Schmelzpunkt von 142 bis 144°C aufweist.

**Patentansprüche**

1. Verfahren zur Herstellung von 3-Alkyl-6-methyl-β-resorcylsäureestern der Formel

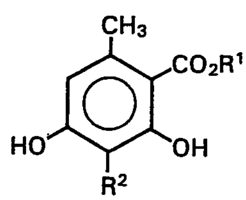

I

in der $R^1$ und $R^2$ gleich oder verschieden sein können und für Alkylgruppen mit 1 bis 10 C-Atomen stehen, dadurch gekennzeichnet, dass man ein α-Pyron der Formel

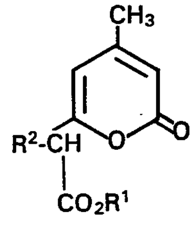

II

in der $R^1$ und $R^2$ die vorstehende Bedeutung haben, mit einer Base umsetzt.

2. Verfahren nach Anspruch 1, dadurch gekennzeichnet, dass man aus den zunächst entstandenen Salzen die freien 3-Alkyl-6-methyl-β-resorcylsäureester durch Ansäuern des Rohansatzes mit Säuren isoliert.

3. Verfahren nach einem der Ansprüche 1 oder 2, dadurch gekennzeichnet, dass als Base Alkoholate, Hydroxide oder Carbonate der Alkalimetalle oder Erdalkalimetalle verwendet werden.

4. Verfahren nach mindestens einem der Ansprüche 1 bis 3, dadurch gekennzeichnet, dass die Reaktion in einem Temperaturbereich von +10°C bis 200°C durchgeführt wird.

5. Verfahren nach mindestens einem der Ansprüche 1 bis 4, dadurch gekennzeichnet, dass die Reaktion in einem Lösungsmittel durchgeführt wird.

**Claims**

1. Process for the production of 3-alkyl-6-methyl-β-resorcylic acid esters of the formula

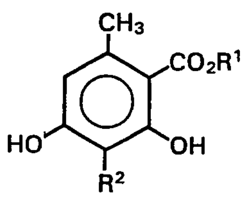

I

in which $R^1$ and $R^2$ can be the same or different and stand for alkyl groups with 1 to 10 C-atoms, characterised in that an α-pyrone of formula

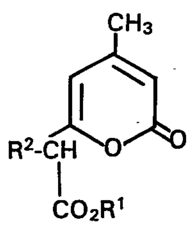

II

in which $R^1$ and $R^2$ have the foregoing meaning, is reacted with a base.

2. Process according to claim 1, characterised in that the free 3-alkyl-6-methyl-β-resorcylic acid ester is isolated from the initially produced salts by acidification of the crude mixture with acids.

3. Process according to one of claims 1 or 2, characterised in that alcoholates, hydroxides or carbonates of alkali metals of alkaline earth metals are used as the base.

4. Process according to at least one of claims 1 to 3, characterised in that the reaction is carried out in a temperature range of +10°C to 200°C.

5. Process according to at least one of claims 1 to 4, characterised in that the reaction is carried out in a solvent.

**Revendications**

1. Procédé de préparation d'esters de l'acide 3-alkyl-6-méthyl-β-résorcylique de formule

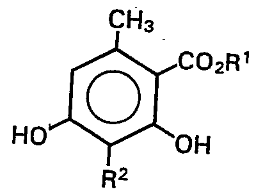

I

dans laquelle $R^1$ et $R^2$ peuvent être identiques ou différents et sont des groupes alkyles avec 1 à 10 atomes de C, caractérisé en ce q'une α-pyrone de formule

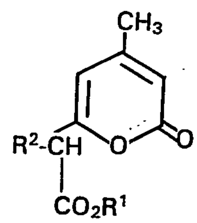

II

dans laquelle $R^1$ et $R^2$ ont la signification précédente, est mise à réagir avec une base.

2. Procédé selon la revendication 1, caractérisé en ce qu'on isole, à partir des sels formés dans un premier temps, les esters d'acide 3-alkyl-6-méthyl-β-résorcylique libres par acidification du mélange brut des acides.

3. Procédé selon l'une des revendications 1 ou 2, caractérisé en ce qu'on utilise comme bases des alcoolates, des hydroxydes ou des carbonates de métaux alcalins ou de métaux alcalino-terreux.

4. Procédé selon au moins l'une des revendications 1 à 3, caractérisé en ce que la réaction est conduite dans un intervalle de température de +10°C à 200°C.

5. Procédé selon au moins d'une des revendications 1 à 4, caractérisé en ce que la réaction a lieu dans un solvant.